(19) **Europäisches Patentamt European Patent Office Office européen des brevets**

(11) **EP 3 700 428 B1**

(12) **EUROPEAN PATENT SPECIFICATION**

(45) Date of publication and mention
of the grant of the patent:
**07.02.2024 Bulletin 2024/06**

(51) International Patent Classification (IPC):
**A61B 8/06** (2006.01)     **A61B 8/08** (2006.01)
**G01S 15/89** (2006.01)     **G01S 7/52** (2006.01)

(21) Application number: **18789082.7**

(52) Cooperative Patent Classification (CPC):
**A61B 8/06; A61B 8/0891; G01S 7/52038;**
**G01S 7/52066; G01S 7/52074; G01S 15/8979;**
A61B 8/483; A61B 8/488; A61B 8/5223

(22) Date of filing: **16.10.2018**

(86) International application number:
**PCT/EP2018/078130**

(87) International publication number:
**WO 2019/081257 (02.05.2019 Gazette 2019/18)**

(54) **ULTRASONIC MEASUREMENT OF VESSEL STENOSIS**

ULTRASCHALLMESSUNG VON GEFÄSSSTENOSE

MESURE ULTRASONORE DE STÉNOSE VASCULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.10.2017 US 201762576235 P**

(43) Date of publication of application:
**02.09.2020 Bulletin 2020/36**

(73) Proprietor: **Koninklijke Philips N.V.**
**5656 AG Eindhoven (NL)**

(72) Inventor: **JAGO, James, Robertson**
**5656 AE Eindhoven (NL)**

(74) Representative: **Philips Intellectual Property &
Standards
High Tech Campus 52
5656 AG Eindhoven (NL)**

(56) References cited:
US-A- 5 606 972          US-A1- 2002 013 530
US-A1- 2003 114 756     US-A1- 2008 287 799
US-A1- 2014 236 011     US-A1- 2015 173 716
US-A1- 2017 215 838

• TEGELER ET AL: "Advances in Carotid
Ultrasound", SEMINARS IN
CEREBROVASCULAR DISEASES AND ST,
SAUNDERS, US, vol. 5, no. 2, 1 June 2005
(2005-06-01), pages 74-82, XP005419159, ISSN:
1528-9931, DOI: 10.1053/J.SCDS.2006.01.012

**Description**

This invention relates to medical diagnostic ultrasound systems and methods, in particular, to the use of ultrasound systems to measure vessel stenosis, the percentage occlusion of a blood vessel.

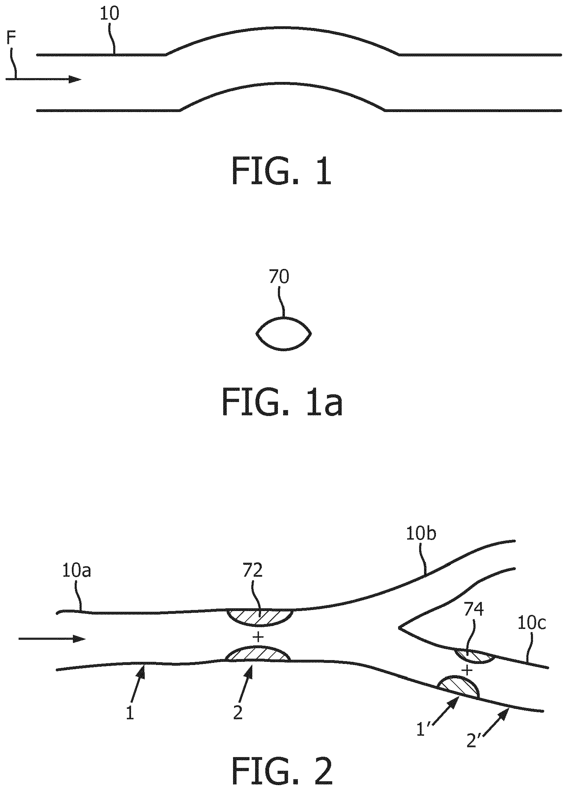

The obstruction of blood vessels by the buildup of plaque and other substances can prevent the flow of an adequate supply of nourishing blood to tissues and organs in the body. Hence it is desirable to be able to detect and measure blood vessel obstructions, generally as a percent stenosis, the percentage reduction of the normal flow lumen caused by the plaque. Visualizing and measuring the obstruction with ultrasound is problematic with two-dimensional (2D) ultrasound due to the difficulty in obtaining the correct image plane for the proper measurement. Three dimensional (D) ultrasound will obviate this problem, but is nonetheless hampered by shadowing from the plaque calcification and insufficient resolution. The most common way to quantify vessel obstruction is not by ultrasound, but by angiography. Since angiograms are projection images, they are useful for assessing vessel diameter reduction and not flow lumen area change. FIGURE 1 illustrates a difficulty in assessing lumen size with projection images. In FIGURE 1 blood is flowing in blood vessel 10 as indicated by flow vector F. Vessel 10 is completely unobstructed in this example, but has a bend as shown in the drawing. If a projection image were taken parallel to the flow direction F, the resultant image of the lumen would appear as shown by lumen 70 in FIGURE 1a. Thus, this view of the vessel 10 could be taken to be that of an obstructed vessel. Angiograms are not normally taken parallel to the flow direction as in FIGURE 1, but normal to the length of the vessel as FIGURE 1 is viewed, but the same principle of reconstruction applies. The resultant angiogram will be strongly affected by the rotational orientation of the plaque within the vessel and the tortuous path of the vessel, and for these reasons numerous angiograms are normally acquired at different look directions to the vessel. By comparing different views, an assessment of the degree of stenosis is made, typically using the NASCET standard which relates the perceived residual lumen diameter at the stenosis to the diameter of the vessel lumen at an unobstructed point in the vessel. Even with multiple views of a vessel, however, degree of stenosis is often underestimated with angiography. Nonetheless, such measurements are preferred over the current ultrasonic method for assessing stenosis, which is to measure the peak blood flow velocity at a stenosis, then relate this velocity to a vessel diameter reduction based on known previous measurements. But ultrasound is simple and easy to use, and does not involve the use of radiographic contrast agents as does angiography. Thus it would be desirable to be able to use ultrasound to perform initial assessment of vessel stenosis if an accurate and reliable ultrasonic technique were available. It would further be desirable for such assessment to measure lumen area reduction rather than diameter reduction, as it has been found that the hemodynamic effects of stenosis are more closely related to residual lumen area rather than diameter.

US 2017/215838 A1 discloses methods and apparatuses for displaying ultrasound images. Methods and apparatuses for displaying ultrasound images determine a stenosis cross-section from ultrasound data with respect to an object including a blood vessel and display at least two cross-sections related to stenosis of the blood vessel and a

stenosis index value representing a degree of stenosis of the blood vessel.

US 2003/114756 A1 discloses an ultrasound imaging method and system including a two-dimensional array transducer scanhead coupled to a beamformer. The beamformer and scanhead obtain signals corresponding to ultrasound echoes reflected from a measurement volume extending across a blood vessel. The signals are processed by a Doppler processor to generate data corresponding to a three-dimensional Doppler image of blood flow velocity in the sample volume. The signals are also processed by a B-mode processor to generate data corresponding to a cross section through the vessel. An image processor transforms the data corresponding to the three-dimensional Doppler image to data corresponding to a projection of the three-dimensional Doppler image onto a plane. The image processor also combines the transformed Doppler data with the B-mode data to create a composite image. Volume flow rate can also be determined by integrating the flow velocity in the projection of the three-dimensional Doppler image.

US 2008/287799 A1 discloses an ultrasound system comprises an ultrasound probe, a user interface and a processor. The ultrasound probe comprises a transducer face emitting ultrasound beams into a patient. The probe acquires a volume of ultrasound data comprising a blood vessel. The user interface defines a surface on an image that is based on the volume. The surface bisects the blood vessel and further comprises a plurality of points where at least some of the points are located at unequal distances with respect to the transducer face. The processor is configured to steer a subset of the ultrasound beams to intersect the surface at a 90-degree angle and calculate volumetric flow information through the blood vessel based on the ultrasound data corresponding to the surface.

US 5606792 A discloses a Doppler signal processor is provided for an ultrasonic diagnostic imaging system which identifies the peak and mean flow velocities by Doppler interrogation of a sample volume within the body. The effects of spectral broadening resulting from the use of an array transducer are compensated by producing a peak velocity value which is a function of the dimension of the array aperture and its relation to the location of the sample volume. Mean velocity values calculated from received Doppler echoes are combined with an array distortion function to produce accurate mean velocity values.

US 2014/0236011 A1 refers to an interventional device to gain structural information of an patients target region using one image sensor and one flow sensor. It is an object of the patent application to estimate functional parameters such as fractional flow reserve, coronary flow reserve or percentage area of stenosis.

In accordance with the principles of the present invention, an ultrasound system including the features of claim 1 and an ultrasonic measurement method including the features of claim 12 are provided. The ultrasound system and the measurement technique are described for measuring the percent stenosis of a vessel in terms of lumen area reduction. A measurement of volume blood flow is made at an unobstructed point of the vessel proximal the site of the obstruction. A measurement of the blood flow velocity is made at the stenosis. The quotient of these two values is computed to produce an estimate of the residual lumen area and the percent stenosis at site of the obstruction. The volume blood flow measurement is preferably made using 3D ultrasound.

In the drawings:

FIGURE 1 illustrates a tortuous unobstructed blood vessel.

FIGURE 1a illustrates a projection image of the lumen of the blood vessel of FIGURE 1 taken in the direction of the blood flow.

FIGURE 2 illustrates a carotid artery with stenotic regions in the common carotid artery and the internal carotid artery which are to be measured for percent stenosis in accordance with the principles of the present invention.

FIGURE 3 illustrates a blood vessel with a cross sectional area where volume blood flow is to be measured.

FIGURE 4 illustrates the measurement of volume blood flow through a virtual surface in front of an ultrasound transducer.

FIGURE 5 illustrates why no angle correction is needed for the volume flow measurement technique of FIGURE 4.

FIGURE 6 illustrates a spectral Doppler display with a tracing of its mean velocity over several heart cycles.

FIGURE 7 is a block diagram of an ultrasound system constructed in accordance with the principles of the present invention.

Referring to FIGURE 2, three sections of a branching carotid artery are illustrated, the common carotid artery 10a, the external carotid artery 10b, and the internal carotid artery 10c. Plaque buildup can occur in the carotid artery, restricting the flow of blood to the brain, and this example illustrates two such areas: an obstruction 72 in the common carotid artery and an obstruction 74 in the internal carotid artery. It is desired to measure the percent stenosis caused by these two obstructions.

In accordance with the principles of the present invention, a volume flow measurement is taken at an unobstructed point in an obstructed artery and a flow velocity measurement is taken at the stenosis. These two values are then used to calculate percent area reduction of the artery caused by the stenosis. These measurements are premised on the fact that volume flow of blood $Q$ through a cross-section of an artery is equal to the time average velocity V of blood flow times the area A of the cross-section, or

$$Q = v \cdot A \qquad [1]$$

In the case of the common carotid artery obstruction, a volume flow measurement is taken at the unobstructed point indicated by the circled "1". At this point in the artery,

$$Q_1 = v_1 \cdot A_1 \qquad [2]$$

where $A_1$ is the unobstructed cross-sectional area at this point in the vessel. Since all of the blood flowing through the vessel at point 1 will then flow through the obstruction at the circled "2", it is known that

$$Q_1 = Q_2 \qquad [3]$$

A time average velocity measurement is now taken at the stenosis at point 2 in the vessel. This may be done using spectral Doppler and measuring the time averaged mean velocity of the blood flow through the stenosis. The user positions a Doppler sample volume cursor over the narrow obstruction of the stenosis as shown by the "+" icon in the drawing, then starts the Doppler acquisition to measure velocity at this point in the vessel. At the stenosis it is known that

$$Q_2 = v_2 \cdot A_2 \qquad [4]$$

where $Q_2$ is the volume flow of blood through the stenotic point 2 and $A_1$ is the area of the residual lumen at the stenosis, the reduced area it is desired to measure. Since it is known that $Q_1 = Q_2$ and the blood flow velocity $v_2$ at the stenosis has been measured by spectral Doppler, the area of the residual lumen is computed by

$$A_2 = \frac{v_2}{Q_2} = \frac{v_2}{Q_1} \qquad [5]$$

and the percent reduction of the area of the lumen of the vessel is

$$100 \, x \left(1 - \frac{A_2}{A_1}\right) \qquad [6]$$

In the internal carotid artery in FIGURE 2, an obstruction is at circled point "1'". The volume flow measurement previously made in the common carotid artery cannot be used to measure the percent stenosis in the internal carotid artery because the blood flow of the CCA splits, with some passing into the ECA and the rest flowing in the ICA. Thus, the volume flow measurement for this second obstruction must be taken in the ICA where all of the blood flowing through the obstruction at point 1' also flow through the vessel at the measurement point, which is the circled "2'" in this example. A volume flow measurement is taken at point 2, and a time average velocity measurement is taken at the stenosis as indicated by the "+" icon. Then the area of the residual lumen at the stenosis is computed as explained above.

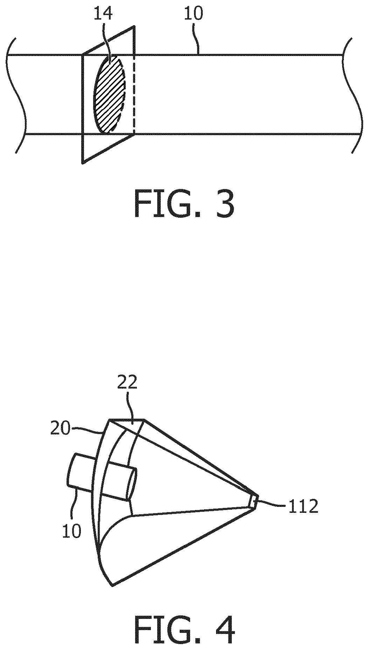

With reference to FIGURE 3, the volume flow rate of blood through a blood vessel 10 can be measured by measuring the volume flow rate through any arbitrary sample surface 14 passing through the vessel. The volume flow rate through the sample surface 14 can be measured by first determining the velocity of blood flowing through the sample surface 14 by performing a three-dimensional Doppler scan. The velocity is then integrated throughout the area of the sample surface 14.

The sample surface 14 can be of any arbitrary shape or orientation. The reason the surface 14 need not be particularly oriented is that whatever volume of blood flows through the vessel 10 also flows through the sample surface 14. Thus, the sample surface 14 can be any arbitrary shape having any arbitrary orientation to the flow of blood through the vessel 10. In a preferred implementation of the present invention, a spherical sample surface 20 is obtained by obtaining a three-dimensional Doppler image in a narrow sample volume 22 equidistant from a two-dimensional array transducer 112 as shown in FIGURE 4. A Doppler scan of this type is in this context referred to as Flow-mode, or F-mode, scanning. A 3-D flow image is obtained by an F-mode scan and is rendered with a spherical cross section 20 through the blood vessel 10, and the velocity values on the virtual spherical surface 20 are integrated to obtain the volume flow measurement as described more fully below.

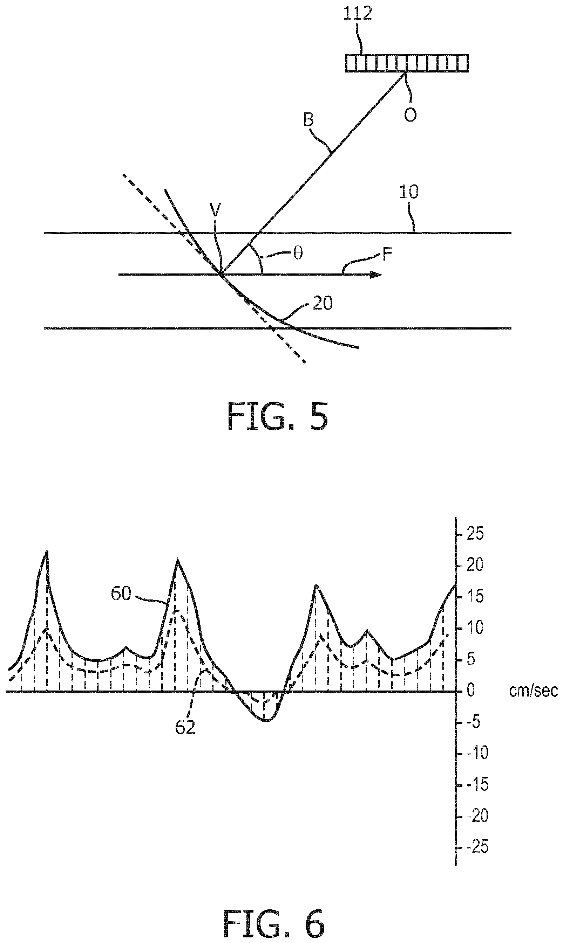

The Doppler flow at points on the virtual spherical surface 20 is sampled by transmitting beams B steered from a common origin O of the two-dimensional array 112 as shown in FIGURE 5. The echo signals at a common depth V along each beam are acquired to thereby acquire echoes on the spherical which intersects the blood vessel 10. The spherical surface is thus normal to the beam at each sampling point V. In instances where the two-dimensional is not square but is rectangular, the virtual surface can be toroidal in shape, but can be used to the same effect. The acquired signals at points V of the beams B will be echoes from blood flow for each point V which is inside the lumen of the blood vessel 20, and will be returned from tissue at points in the vessel wall and surrounding tissue. The flow signal can therefore be segmented by a Doppler wall filter as is known in the art. To account for boundary effects where echoes are returned from points near the vessel wall, and are thus likely to be a mix of flow and tissue signals, the returning echoes can be weighted by the intensity of the power Doppler characteristic of each echo, thereby weighting signals from the lumen boundary less than those more in the interior of the vessel. Normally, the measured Doppler velocity values on the surface are angle-dependent and need to be scaled as a function of the cosine of the incident Doppler angle, the angle between the Doppler beam B and the direction of flow F. But since the Doppler beam B is perpendicular to the unit area of the surface 20 at the sampling point V, as indicated by the dashed line demarcating the plane of the unit area, the angle between the perpendicular to the unit area and the flow direction has the same cosine term as the Doppler angle θ. Thus, Gauss's law for volume flow results in cancellation of the two cosine terms and no scaling of the measured velocity values is needed prior to summation (integration) of the velocity values in the lumen.

FIGURE 6 illustrates a typical spectral Doppler display produced by an ultrasound system. The abscissa is calibrated in cm/sec and the ordinate is a time axis. Each vertical line is a measure of the spread of velocities at the sample volume in the subject from which the Doppler signals are acquired, e.g., the + icon in FIGURE 3, at the time of acquisition. The peak velocity values are traced from one spectral line to the next by a trace 60, and the mean velocity values are connected by a dashed line 62. The acquisition and display of the Doppler spectrum of FIGURE 6 is detailed in US Pat.5,606,972 (Routh). In an implementation of the present invention it is preferred to use a time averaged mean velocity value for the blood flow velocity value at a stenosis, which is obtained by averaging the mean velocity values on dashed line 62 over the interval of a heart cycle.

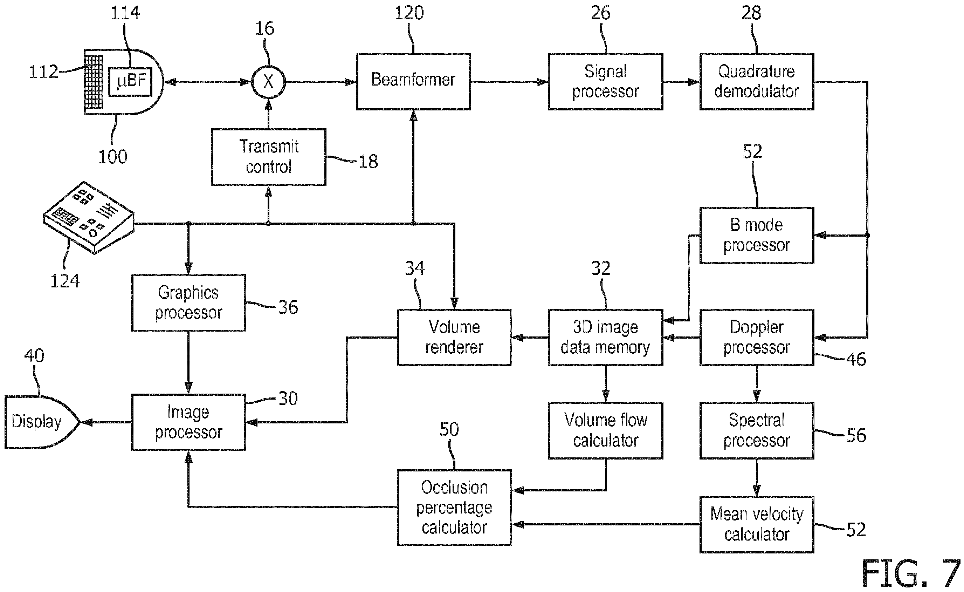

Referring to FIGURE 7, an ultrasound system constructed for measuring the area reduction of a vessel due to a stenosis in accordance with the present invention is shown in block diagram form. A transducer array 112 is provided in an ultrasound probe 100 for transmitting ultrasonic waves and receiving echo information over a volumetric region of a body. The transducer array 112 may be a two-dimensional array of transducer elements capable of electronically scanning in two or three dimensions, in both elevation (in 3D) and azimuth, as shown in the drawing. Alternatively, the transducer may be a one-dimensional array of elements capable of scanning image planes which is oscillated back and forth to sweep the image plane through a volumetric region and thereby scan the region for three-dimensional imaging, such as that described in US Pat. 7,497,830 (Li et al.) A two-dimensional transducer array 112 is coupled to a microbeamformer 114 in the probe which controls transmission and reception of signals by the array elements. Microbeamformers are capable of at least partial beamforming of the signals received by groups or "patches" of transducer elements as described in US Pats. 5,997,479 (Savord et al.), 6,013,032 (Savord), and 6,623,432 (Powers et al.) The microbeamformer is coupled by the probe cable to a transmit/receive (T/R) switch 16 which switches between transmission and reception and protects the main system beamformer 120 from high energy transmit signals. The transmission of ultrasonic beams from the transducer array 112 under control of the microbeamformer 114 is directed by a transmit controller 18 coupled to the T/R switch and the beamformer 120, which receives input from the user's operation of the ultrasound system user interface or controls 124. Among the transmit characteristics controlled by the transmit controller are the spacing, amplitude, phase, and polarity of transmit beams and waveforms. Beams formed in the direction of pulse transmission may be steered straight ahead from the transducer array, or at different angles for a wider sector field of view or to scan a volumetric region such as that in front of transducer array 112 and including spherical surface 20 in FIGURE 4.

The echoes received by a contiguous group of transducer elements are beamformed by appropriately delaying them and then combining them. The partially beamformed signals produced by the microbeamformer 114 from each patch of transducer elements are coupled to a main beamformer 120 where partially beamformed signals from individual patches of transducer elements are delayed and combined into a fully beamformed coherent echo signal. For example, the main beamformer 120 may have 128 channels, each of which receives a partially beamformed signal from a patch of 12 transducer elements. In this way the signals received by over 1500 transducer elements of a two-dimensional array transducer can contribute efficiently to a single beamformed signal.

The coherent echo signals undergo signal processing by a signal processor 26, which includes filtering by a digital filter and noise reduction as by spatial or frequency compounding. The digital filter of the signal processor 26 can be a filter of the type disclosed in U.S. Patent No. 5,833,613 (Averkiou et al.), for example. The processed echo signals are demodulated into quadrature (I and Q) components by a quadrature demodulator 28, which provides signal phase information and can also shift the signal information to a baseband range of frequencies.

The beamformed and processed coherent echo signals are coupled to a B mode processor 52 which produces a B mode image of structure in the body such as tissue. The B mode processor performs amplitude (envelope) detection of quadrature demodulated I and Q signal components by calculating the echo signal amplitude in the form of $(I^2+Q^2)^{1/2}$. The quadrature echo signal components are also coupled to a Doppler processor 46, which stores ensembles of echo signals from discrete points in an image field which are then used to estimate the Doppler shift at points in the image, *e.g.,* the points on a virtual spherical surface intersecting a blood vessel, with a fast Fourier transform (FFT) processor. The Doppler shift is proportional to motion at points in the image field, *e.g.,* blood flow and tissue motion. For a color Doppler image, a surface of which may be used for the volume flow measurement, the estimated Doppler flow values at each point on the virtual surface 20 through a blood vessel are wall filtered and the surface Doppler values used to produce the volume flow measurement as described above. The surface Doppler values and others throughout a scanned volume may also be converted to color values using a look-up table to produce a colorflow Doppler image. Either the B mode image or the Doppler image may be displayed alone, or the two shown together in anatomical registration in which the color Doppler overlay shows the blood flow in tissue and in vessels in the imaged region.

The B mode image signals and the Doppler flow values are coupled to a 3D image data memory 32, which stores the image data in x, y, and z addressable memory locations corresponding to spatial locations in a scanned volumetric region of a subject. This volumetric image data is coupled to a volume renderer 34 which converts the echo signals of a 3D data set into a projected 3D image as viewed from a given reference point as described in US Pat. 6,530,885 (Entrekin et al.) The reference point, the perspective from which the imaged volume is viewed, may be changed by manipulation of a control on the control panel 124, which enables the volume to be tilted or rotated to observe the scanned region from different viewpoints. The volume rendered image is coupled to an image processor 30 for display on a display 40.

In accordance with the principles of the present invention, the Doppler signal samples acquired from the sample volume + at the stenosis are coupled to a spectral Doppler display processor 56. The mean velocity values traced on each spectral line as shown in FIGURE 6 are averaged over the interval of a heart cycle by a mean velocity calculator 52 to produce a time averaged mean velocity value of the blood flow at the stenosis which is coupled to an occlusion percentage calculator 50. The Doppler flow velocity values acquired at points on the virtual surface 20 which are stored in the 3D image data memory 32 are coupled to a volume flow calculator 54 which sums (integrates) the velocity values to produce a volume flow value, which is coupled to the occlusion percentage calculator. The occlusion percentage calculator computes the quotient of the time averaged mean velocity value at the stenosis and the volume flow measurement at the unoccluded point in the vessel to compute the residual flow lumen area at the stenosis using equation [5] above. The percent area reduction (occlusion percentage) may also be computed by the occlusion percentage calculator using equation [6]. The area $A_1$ of the unoccluded lumen, area 14 in FIGURE 3, may be calculated by segmenting out the lumen area normal to the direction of flow from the colorflow volume image by multiplanar reconstruction, and measuring the area using known techniques, as ultrasound image data is calibrated to be anatomically accurate. Alternately, $A_1$ may be calculated by making one or more velocity measurements at the unoccluded point in the lumen to compute $v_1$ and using the volume flow measurement to compute $A_1$ using equation [2]. The residual lumen area at the stenosis and/or the percentage stenosis values are coupled to the image processor or a graphics processor 36 for display on image display 40. The graphics processor may also be employed if desired to illustrate the virtual surface 20 in registration with the 3D ultrasound image on the display.

It should be noted that an ultrasound system suitable for use in an implementation of the present invention, and in particular the component structure of the ultrasound system of FIGURE 7, may be implemented in hardware, software or a combination thereof. The various embodiments and/or components of an ultrasound system, for example, the processors, calculators, and volume renderer of FIGURE 7, or components, processors, and controllers therein, also may be implemented as part of one or more computers or microprocessors. The computer or processor may include a computing device, an input device, a display unit and an interface, for example, for accessing the Internet. The computer or processor may include a microprocessor. The microprocessor may be connected to a communication bus, for example, to access a PACS system or the data network for importing training images. The computer or processor may also include a memory. The memory devices such as the 3D image data memory and those used to store Doppler ensembles may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including those controlling the acquisition, processing, and transmission of ultrasound images as described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules such as a neural network model module, a program module within a larger program or a portion of a program module. The software also may include modular programming in the form of object-oriented programming. The processing of input data by the processing machine may be in response to operator commands, or in response to results of previous processing, or in response to a request made by another processing machine.

## Claims

1. An ultrasonic diagnostic imaging system for assessing the degree of stenosis of a vessel (10) caused by an obstruction, the ultrasonic diagnostic imaging system comprising:

   an ultrasound probe (100) adapted to acquire three-dimensional ultrasound data from blood flow in the vessel (10);
   a 3D data memory (32) coupled to the ultrasound probe (100), and adapted to store the three-dimensional ultrasound data from blood flow in the vessel (10);
   a volume flow calculator (54), coupled to the 3D data memory (32), and adapted to compute a volume flow measurement at an unobstructed point of the vessel (10) ;
   a Doppler processor (46), coupled to the ultrasound probe (100) and responsive to ultrasound data from blood flow in the vessel (10), and adapted to produce a velocity measurement at the stenosis of the vessel (10); and
   an occlusion calculator (50), responsive to the volume flow measurement and the velocity measurement, and adapted to produce a measurement of the flow reduction caused by the stenosis based on a quotient of the velocity measurement at the stenosis of the vessel (10) and the volume flow measurement at the unobstructed point of the vessel (10),
   wherein the volume flow calculator (54) is further adapted to calculate the sum or integral of velocity values of a virtual surface (20) intersecting the vessel (10), and
   wherein the virtual surface (20) is obtained by obtaining a three-dimensional Doppler image in a narrow sample volume (22) equidistant from a two-dimensional array transducer (112) accommodated in the probe (100).

2. The ultrasonic diagnostic imaging system of Claim 1, wherein the occlusion calculator (50) is further adapted to produce a measurement of the degree of stenosis of the vessel (10).

3. The ultrasonic diagnostic imaging system of Claim 1, wherein the occlusion calculator (50) is further adapted to produce a measurement of the percent reduction of the area of a lumen of the vessel (10) caused by the stenosis.

4. The ultrasonic diagnostic imaging system of Claim 1, wherein the occlusion calculator (50) is further adapted to produce a measurement of the area of a residual lumen of the vessel (10).

5. The ultrasonic diagnostic imaging system of Claim 1, wherein the virtual surface (20) further comprises a spherical virtual surface.

6. The ultrasonic diagnostic imaging system of Claim 1, wherein the virtual surface (20) further comprises a toroidal virtual surface.

7. The ultrasonic diagnostic imaging system of Claim 1, wherein the volume flow calculator (54) is further adapted to calculate the sum or integral of Doppler velocity values located on a virtual surface (20) intersecting the vessel (10).

8. The ultrasonic diagnostic imaging system of Claim 1, wherein the velocity values are weighted in proportion to power Doppler values calculated for locations corresponding to locations of the velocity values in the vessel (10).

9. The ultrasonic diagnostic imaging system of Claim 1, wherein the Doppler processor (46) further comprises a spectral Doppler processor.

10. The ultrasonic diagnostic imaging system of Claim 9, wherein the spectral Doppler processor further comprises a time averaged mean velocity calculator.

11. The ultrasonic diagnostic imaging system of Claim 10, wherein the occlusion calculator (50) is further adapted to compute the quotient of a time averaged mean velocity value and a volume flow measurement.

12. A method for ultrasonically measuring the degree of stenosis at a point in a vessel (10) comprising:

measuring volume flow at an unoccluded point in the vessel (10);
measuring flow velocity at an occluded point in the vessel (10);
computing area of stenosis at the occluded point using the volume flow measurement and the flow velocity measurement,
calculating the sum or integral of velocity values of a virtual surface (20) intersecting the vessel (10),
wherein the virtual surface (20) is obtained by obtaining a three-dimensional Doppler image in a narrow sample volume (22) equidistant from a two-dimensional array transducer (112) accommodated in the probe (100).

13. The method of Claim 12, wherein measuring volume flow further comprises summing or integrating velocity values of a virtual surface intersecting the vessel (10) .

14. The method of Claim 12, wherein measuring flow velocity further comprises measuring time averaged mean velocity at the occluded point by spectral Doppler analysis.

**Patentansprüche**

1. Diagnostisches Ultraschall-Bildgebungssystem zur Beurteilung des Stenosegrades eines Gefäßes (10), der durch eine Obstruktion verursacht wird, wobei das diagnostische Ultraschall-Bildgebungssystem Folgendes umfasst:

eine Ultraschallsonde (100), die so angepasst ist, dass sie dreidimensionale Ultraschalldaten vom Blutfluss im Gefäß (10) erfasst;
einen 3D-Datenspeicher (32), der mit der Ultraschallsonde (100) gekoppelt und zum Speichern der dreidimensionalen Ultraschalldaten vom Blutfluss im Gefäß (10) angepasst ist;
einen Volumenstromrechner (54), der mit dem 3D-Datenspeicher (32) verbunden ist und dazu angepasst ist, eine Volumenstrommessung an einem freien Punkt des Gefäßes (10) zu berechnen;
einen Doppler-Prozessor (46), der mit der Ultraschallsonde (100) gekoppelt ist und auf Ultraschalldaten vom Blutfluss im Gefäß (10) anspricht und so angepasst ist, dass er eine Geschwindigkeitsmessung an der Stenose des Gefäßes (10) erzeugt; und
einen Okklusionsrechner (50), der auf die Volumenstrommessung und die Geschwindigkeitsmessung anspricht und so angepasst ist, dass er eine Messung der durch die Stenose verursachten Flussreduzierung basierend auf einem Quotienten aus der Geschwindigkeitsmessung an der Stenose des Gefäßes (10) und der Volumenstrommessung an der freien Stelle des Gefäßes (10) erzeugt,

wobei der Volumenstromrechner (54) außerdem dazu angepasst ist, die Summe oder das Integral von Geschwindigkeitswerten einer virtuellen Oberfläche (20) zu berechnen, die das Gefäß (10) schneidet, und

wobei die virtuelle Oberfläche (20) durch Erhalten eines dreidimensionalen Dopplerbildes in einem schmalen Probenvolumen (22) mit gleichem Abstand von einem zweidimensionalen Array-Wandler (112), der in der Sonde (100) untergebracht ist, erhalten wird.

2. Diagnostisches Ultraschall-Bildgebungssystem nach Anspruch 1, wobei der Okklusionsrechner (50) außerdem dazu angepasst ist, eine Messung des Stenosegrades des Gefäßes (10) zu erzeugen.

3. Diagnostisches Ultraschall-Bildgebungssystem nach Anspruch 1, wobei der Okklusionsrechner (50) außerdem dazu angepasst ist, eine Messung der prozentualen Verringerung der Fläche eines Lumens des Gefäßes (10) zu erzeugen, die durch die Stenose verursacht wird.

4. Diagnostisches Ultraschall-Bildgebungssystem nach Anspruch 1, wobei der Okklusionsrechner (50) außerdem dazu angepasst ist, eine Messung der Fläche eines Restlumens des Gefäßes (10) zu erzeugen.

5. Diagnostisches Ultraschall-Bildgebungssystem nach Anspruch 1, wobei die virtuelle Oberfläche (20) außerdem eine sphärische virtuelle Oberfläche umfasst.

6. Diagnostisches Ultraschall-Bildgebungssystem nach Anspruch 1, wobei die virtuelle Oberfläche (20) außerdem eine ringförmige virtuelle Oberfläche umfasst.

7. Diagnostisches Ultraschall-Bildgebungssystem nach Anspruch 1, wobei der Volumenstromrechner (54) außerdem dazu angepasst ist, die Summe oder das Integral von Doppler-Geschwindigkeitswerten zu berechnen, die sich auf einer virtuellen Oberfläche (20) befinden, die das Gefäß (10) schneidet.

8. Diagnostisches Ultraschall-Bildgebungssystem nach Anspruch 1, wobei die Geschwindigkeitswerte im Verhältnis zu Leistungs-Doppler-Werte gewichtet werden, die für Orte berechnet werden, die den Orten der Geschwindigkeitswerte im Gefäß (10) entsprechen.

9. Diagnostisches Ultraschall-Bildgebungssystem nach Anspruch 1, wobei der Doppler-Prozessor (46) außerdem einen Spektral-Doppler-Prozessor umfasst.

10. Diagnostisches Ultraschall-Bildgebungssystem nach Anspruch 9, wobei der Spektral-Doppler-Prozessor außerdem einen zeitlich gemittelten mittleren Geschwindigkeitsrechner umfasst.

11. Diagnostisches Ultraschall-Bildgebungssystem nach Anspruch 10, wobei der Okklusionsrechner (50) außerdem dazu angepasst ist, den Quotienten aus einem zeitlich gemittelten mittleren Geschwindigkeitswert und einer Volumenstrommessung zu berechnen.

12. Verfahren zur Ultraschallmessung des Stenosegrades an einer Stelle in einem Gefäß (10), umfassend:

Messen des Volumenstroms an einem nicht verschlossenen Punkt im Gefäß (10);
Messen der Strömungsgeschwindigkeit an einem verschlossenen Punkt im Gefäß (10);
Berechnen des Stenosebereichs am verschlossenen Punkt unter Verwendung der Volumenstrommessung und der Strömungsgeschwindigkeitsmessung,
Berechnen der Summe oder des Integrals von Geschwindigkeitswerten einer virtuellen Oberfläche (20), die das Gefäß (10) schneidet,
wobei die virtuelle Oberfläche (20) durch Erhalten eines dreidimensionalen Dopplerbildes in einem schmalen Probenvolumen (22) mit gleichem Abstand von einem zweidimensionalen Array-Wandler (112), der in der Sonde (100) untergebracht ist, erhalten wird.

13. Verfahren nach Anspruch 12, wobei das Messen des Volumenstroms außerdem das Summieren oder Integrieren von Geschwindigkeitswerten einer virtuellen Oberfläche umfasst, die das Gefäß (10) schneidet.

14. Verfahren nach Anspruch 12, wobei das Messen der Strömungsgeschwindigkeit außerdem das Messen der zeitlich gemittelten mittleren Geschwindigkeit am verschlossenen Punkt durch Spektral-Doppler-Analyse umfasst.

**Revendications**

1. Système d'imagerie diagnostique ultrasonique pour évaluer le degré de sténose d'un vaisseau (10) provoqué par une obstruction, le système d'imagerie diagnostique ultrasonore comprenant:

une sonde ultrasonore (100) adaptée pour acquérir des données ultrasonores tridimensionnelles à partir du flux sanguin dans le vaisseau (10);

une mémoire de données 3D (32) couplée à la sonde ultrasonore (100), et adaptée pour stocker les données ultrasonores tridimensionnelles provenant du flux sanguin dans le vaisseau (10);

un calculateur de flux volumique (54), couplé à la mémoire de données 3D (32), et adapté pour calculer une mesure de flux volumique en un point non obstrué du vaisseau (10);

un processeur Doppler (46), couplé à la sonde ultrasonore (100) et sensible aux données ultrasonores provenant du flux sanguin dans le vaisseau (10), et adapté pour produire une mesure de vitesse au niveau de la sténose du vaisseau (10); et

un calculateur d'occlusion (50), sensible à la mesure du flux volumique et à la mesure de la vitesse, et adapté pour produire une mesure de la réduction du flux provoquée par la sténose sur la base d'un quotient de la mesure de la vitesse au niveau de la sténose du vaisseau (10) et de la mesure du flux volumique au niveau du point non obstrué du vaisseau (10),

où le calculateur de flux volumique (54) est en outre adapté pour calculer la somme ou l'intégrale des valeurs de vitesse d'une surface virtuelle (20) coupant le vaisseau (10), et

où la surface virtuelle (20) est obtenue en obtenant une image Doppler tridimensionnelle dans un volume d'échantillon étroit (22) équidistant d'un transducteur à réseau bidimensionnel (112) logé dans la sonde (100).

2. Système d'imagerie diagnostique ultrasonique selon la revendication 1, dans lequel le calculateur d'occlusion (50) est en outre adapté pour produire une mesure du degré de sténose du vaisseau (10).

3. Système d'imagerie diagnostique ultrasonique selon la revendication 1, dans lequel le calculateur d'occlusion (50) est en outre adapté pour produire une mesure du pourcentage de réduction de la surface d'un lumen du vaisseau (10) provoquée par la sténose.

4. Système d'imagerie diagnostique ultrasonique selon la revendication 1, dans lequel le calculateur d'occlusion (50) est en outre adapté pour produire une mesure de la surface d'un lumen résiduel du vaisseau (10) .

5. Système d'imagerie diagnostique ultrasonique selon la revendication 1, dans lequel la surface virtuelle (20) comprend en outre une surface virtuelle sphérique.

6. Système d'imagerie diagnostique ultrasonique selon la revendication 1, dans lequel la surface virtuelle (20) comprend en outre une surface virtuelle toroïdale.

7. Système d'imagerie diagnostique ultrasonique selon la revendication 1, dans lequel le calculateur de flux volumique (54) est en outre adapté pour calculer la somme ou l'intégrale des valeurs de vitesse Doppler qui se trouvent sur une surface virtuelle (20) coupant le vaisseau (10).

8. Système d'imagerie diagnostique ultrasonique selon la revendication 1, dans lequel les valeurs de vitesse sont pondérées proportionnellement aux valeurs de puissance Doppler calculées pour des emplacements correspondant aux emplacements des valeurs de vitesse dans le vaisseau (10).

9. Système d'imagerie diagnostique ultrasonique selon la revendication 1, dans lequel le processeur Doppler (46) comprend en outre un processeur Doppler spectral.

10. Système d'imagerie diagnostique ultrasonique selon la revendication 9, dans lequel le processeur Doppler spectral comprend en outre un calculateur de vitesse moyenne calculée au fil du temps.

11. Système d'imagerie diagnostique ultrasonique selon la revendication 10, dans lequel le calculateur d'occlusion (50) est en outre adapté pour calculer le quotient d'une valeur de vitesse moyenne calculée au fil du temps et d'une mesure de flux volumique.

12. Procédé pour mesurer par ultrasons le degré de sténose en un point d'un vaisseau (10) consistant à:

mesurer le flux volumique en un point non occlus dans le vaisseau (10);

mesurer la vitesse de flux en un point occlus dans le vaisseau (10);

calculer la zone de sténose au niveau du point occlus en utilisant la mesure du flux volumique et la mesure de la vitesse de flux,

calculer la somme ou l'intégrale des valeurs de vitesse d'une surface virtuelle (20) coupant le vaisseau (10),

où la surface virtuelle (20) est obtenue en obtenant une image Doppler tridimensionnelle dans un volume d'échantillon étroit (22) équidistant d'un transducteur à réseau bidimensionnel (112) logé dans la sonde (100).

13. Procédé selon la revendication 12, dans lequel la mesure du flux de volume comprend en outre la somme ou l'intégration des valeurs de vitesse d'une surface virtuelle coupant le vaisseau (10).

**14.** Procédé selon la revendication 12, dans lequel la mesure du flux de vitesse comprend en outre la mesure de la vitesse moyenne calculée au fil du temps au niveau du point occlus par analyse Doppler spectrale.

FIG. 1

FIG. 1a

FIG. 2

FIG. 3

FIG. 4

FIG. 5

FIG. 6

FIG. 7

**EP 3 700 428 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2017215838 A1
- US 2003114756 A1
- US 2008287799 A1
- US 5606792 A
- US 20140236011 A1
- US 5606972 A, Routh
- US 7497830 B, Li
- US 5997479 A, Savord
- US 6013032 A, Savord
- US 6623432 B, Powers
- US 5833613 A, Averkiou
- US 6530885 B, Entrekin